# FASCICULE DE BREVET EUROPEEN

(11) **EP 3 096 819 B1**
(45) Date de publication et mention de la délivrance du brevet: **06.06.2018**
(21) Numéro de dépôt: 15700691.7
(22) Date de dépôt: 19.01.2015
(51) Int. Cl.: A61M 5/315, A61M 5/32, A61M 5/20

(54) **INJECTEUR AUTOMATIQUE**
AUTOMATISCHER INJEKTOR
AUTOMATIC INJECTOR

(30) Priorité: 20.01.2014 FR 1450417
(43) Date de publication de la demande: 30.11.2016
(73) Titulaire: Biocorp Production, 63500 Issoire (FR)
(72) Inventeur: ANEAS, Antoine, 63200 Menetrol (FR)
(74) Mandataire: Lavoix
(86) Numéro de dépôt international: PCT/EP2015/050848
(87) Numéro de publication internationale: WO 2015/107180

(56) Documents cités:
- EP-A1- 2 438 947
- EP-A1- 2 468 329
- EP-A2- 0 666 084
- WO-A1-2010/035060

## Description

La présente invention concerne un injecteur automatique, ou auto-injecteur.

Dans le domaine médical, les médicaments pour le traitement d'urgence sont stockés dans des seringues en verre équipées d'une aiguille collée, d'un piston et d'un protège aiguille. Ces seringues sont généralement insérées dans le corps d'un auto-injecteur, pour faciliter l'administration du médicament.

Les auto-injecteurs sont particulièrement adaptés aux situations d'urgence comme une intoxication au gaz, une crise d'allergie grave ou encore un malaise cardiaque. En effet, certains militaires utilisent des auto-injecteurs en cas d'attaque chimique pour s'administrer une dose d'atropine. De même, les auto-injecteurs sont souvent utilisés pour injecter de l'adrénaline dans le corps d'un patient faisant un malaise cardiaque ou une crise d'allergie.

Un auto-injecteur comprend un boitier globalement tubulaire, qui contient la seringue renfermant le médicament. L'injection du médicament dans le corps du patient est obtenue au moyen d'un premier ressort qui se détend pour pousser un piston dans la seringue. En pratique, l'auto-injecteur est muni d'une tige de poussée du piston, qui est retenue par des moyens de blocage.

FR-A-2 654 938 divulgue un auto-injecteur dans lequel ces moyens de blocage sont formés par une pince de retenue de la tige, comprenant des doigts élastiques enserrant la tige. Les doigts de la pince peuvent être écartés de la tige en utilisant un dispositif à coin et l'effort transmis aux doigts provient d'un bouton poussoir disposé à l'arrière de l'auto-injecteur. L'écartement des doigts de la pince permet de relâcher l'effort de serrage de la pince sur la tige, ce qui libère le mouvement d'avancée du piston sous l'action de charge élastique du premier ressort.

FR-A-2 733 155 et WO-A-94/11041 divulguent chacun un auto-injecteur dans lequel les moyens de blocage de l'avancée du piston comprennent un anneau élastique de retenue, qui est disposé autour de la tige et qui présente un diamètre intérieur inférieur au diamètre maximal de la tige. Ces moyens de blocage comprennent en outre un manchon extérieur, qui est mobile selon une direction parallèle à l'axe de déplacement de la tige, entre une position verrouillée, où il est disposé radialement autour de l'anneau élastique et où il empêche l'expansion radiale de l'anneau élastique sous l'effort de charge élastique du premier ressort, et une position reculée, où il n'empêche pas la déformation élastique de l'anneau. De cette manière, la tige ne peut pas passer à travers l'anneau tant que le manchon n'est pas reculé et le mouvement du piston à l'intérieur de la seringue est bloqué. Dans FR-A-2 733 155, le manchon est chargé élastiquement par un ressort dans sa position verrouillée et peut être reculé par l'utilisateur pour libérer la tige, notamment au moyen d'un cran extérieur. Dans WO-A-94/11041, le manchon de guidage est chargé élastiquement par un ressort vers sa position reculée et est maintenu en position par un pion. Ce pion peut être retiré en décollant une languette, ou goupille, de manière à libérer la détente du ressort agissant sur le manchon.

EP-A-0 666 084 divulgue un auto-injecteur selon le préambule de la revendication 1, dans lequel les moyens de blocage de l'avancée du piston comprennent un loquet, qui est engagé dans une encoche de la tige. Ce loquet peut être dégagé de son encoche en appuyant sur un bouton transversal du stylo. Ainsi, il libère le mouvement d'avancée de la tige. Par ailleurs, cet auto-injecteur comporte un poussoir, ou embout de protection de l'aiguille, qui est reculé au contact de l'épiderme à l'encontre de l'action de charge élastique d'un deuxième ressort. Ainsi, lorsque l'auto-injecteur est retiré du corps du patient, l'embout recouvre l'aiguille sous l'action de charge élastique du deuxième ressort. Néanmoins, ce mouvement de recouvrement n'entraine pas l'arrêt du déplacement du piston, c'est à dire que l'injection n'est pas arrêtée.

Le problème des auto-injecteurs précités est que, lorsque la tige de poussée du piston est libérée, l'injection s'effectue d'une seule traite. Or, les militaires intoxiqués au gaz ou les patients en état de stress peuvent trembler au moment de l'injection et retirer accidentellement l'injecteur de la partie de leur corps visée, si bien qu'ils n'arrivent pas à injecter la totalité du médicament. Ainsi, si l'utilisateur retire, pour une raison quelconque, l'auto-injecteur de son corps, l'injection du principe actif se poursuit dans le vide. L'injection est donc incomplète et une certaine quantité de médicament est perdue.
Ce problème est résolu dans EP-A-2 438 947 et EP-A-2 468 329.

Dans EP-A-2 438 947, la seringue est maintenue dans un support mobile à l'intérieur du capot. L'auto-injecteur comprend une tige apte à pousser successivement la seringue et le piston de la seringue sous l'effort élastique d'un ressort et un embout de protection de l'aiguille, qui est mobile axialement à l'encontre d'un effort élastique généré par un ressort.

Lorsque l'auto-injecteur est pressé contre l'épiderme d'un patient, l'embout de protection est repoussé en position distale. En maintenant ou en accentuant la pression de l'auto-injecteur sur l'épiderme, la seringue est déplacée en direction proximale, de sorte que l'aguille creuse pénètre à l'intérieur de l'épiderme du patient. Dans un deuxième temps, la tige pousse le piston dans la direction proximale, ce qui permet d'éjecter le médicament de la seringue.

Dans EP-A-2 468 329, un manchon de déverrouillage est disposé autour du capot extérieur et un tube intérieur, fileté extérieurement, est agencé dans un tube extérieur taraudé complémentaire. Le tube extérieur est soumis à un couple élastique généré par un ressort chargé en torsion. La rotation du tube entraîne l'avancée du tube intérieur et le déplacement en translation d'un piston interne de la seringue. Une roue est montée à l'extrémité du tube extérieur. Cette roue est équipée de dents externes qui coopèrent avec des ergots élastiques internes du capot extérieur pour empêcher la rotation du tube extérieur sous le moment élastique du ressort. Le manchon de déverrouillage comporte un rebord axial qui s'oppose au basculement des ergots vers l'extérieur. Lorsque l'auto-injecteur est pressé contre l'épiderme d'un patient, le manchon de déverrouillage se déplace axialement vers l'avant et libère les ergots, de manière que les ergots peuvent basculer radialement vers l'extérieur et libérer le mouvement de rotation de la roue, c'est-à-dire du manchon extérieur, sous le moment élastique généré par le ressort. Le tube avance alors vers l'avant, ce qui a pour effet de pousser, d'une part, la seringue vers l'avant de manière que l'aiguille creuse pénètre dans l'épiderme du patient et, d'autre part, le piston interne à l'intérieur du corps de seringue de manière que le médicament est éjecté à travers l'aiguille creuse dans le corps du patient.

Dans EP-A-2 438 947 et EP-A-2 468 329, l'étape de piqûre est entièrement automatisée en déplaçant le corps de seringue pour faire pénétrer l'aiguille dans l'épiderme. Ce fonctionnement automatique peut surprendre un utilisateur non averti car il n'est pas conventionnel et peut mener, de ce fait, à une piqûre mal exécutée.

C'est à ces inconvénients qu'entend plus particulièrement remédier l'invention en proposant un injecteur automatique avec lequel l'étape de piqûre reste manuelle et avec lequel l'injection du principe actif est stoppée lorsque l'injecteur est retiré pendant l'injection et avec lequel l'injection peut reprendre si l'utilisateur presse à nouveau l'injecteur sur son corps.

A cet effet, l'invention concerne un injecteur automatique qui comprend un capot, s'étendant selon un axe longitudinal, une seringue d'injection d'un médicament, qui est disposée à l'intérieur du capot et qui comporte une aiguille, un protège-aiguille, un corps et un piston, une tige de poussée du piston à l'intérieur du corps de seringue, qui est déplacée axialement vers l'avant par un effort de charge élastique exercé par un premier ressort, lors d'une injection, un embout de protection de l'aiguille, qui est déplaçable axialement autour de l'aiguille et à l'encontre d'un effort de charge élastique exercé par un deuxième ressort, entre une position avancée où il entoure l'aiguille et une position reculée, où l'aiguille est découverte, et des moyens de blocage de l'avancée de la tige. Les moyens de blocage sont configurés pour être désactivés lorsque l'embout est déplacé vers sa position reculée ou parvient dans cette position, et pour être réactivés en cours d'injection lorsque l'embout est déplacé vers sa position avancée ou parvient dans cette position. Les moyens de blocage de l'avancée de la tige peuvent comprendre un premier levier de blocage du déplacement de la tige, qui est prévu pour bloquer l'avancée d'une crémaillère ménagée sur la tige de poussée. L'embout comprend alors une came de libération de l'action de blocage du premier levier sur la crémaillère, cette came entrainant le basculement du premier levier vers une configuration dégagée de la crémaillère lorsque l'embout parvient en position reculée. En variante, les moyens de blocage de l'avancée de la tige comprennent deux ailettes élastiques, qui appartiennent à l'embout et qui sont disposées de part et d'autre de la tige de poussée, et deux butées de bocage des ailettes élastiques dans une position où les ailettes élastiques maintiennent entre elles la tige en étau.

Grâce à l'invention, si pour une raison quelconque, l'utilisateur retire l'injecteur de sa peau, c'est-à-dire relâche partiellement ou totalement la pression de l'injecteur au contact de la peau, l'embout de protection de l'aiguille revient vers sa position reculée et les moyens de blocage de l'avancée de la tige de poussée du piston à l'intérieur du corps de la seringue sont réactivés. Ainsi, l'injection de médicament est stoppée et il n'y a pas de perte de médicament lorsque l'utilisateur retire l'injecteur de sa peau avant la fin de l'injection. En outre, l'utilisateur peut terminer son injection en appliquant à nouveau l'injecteur sur une partie de son corps puisque cela entraine à nouveau le déplacement de l'embout vers sa position reculée et la désactivation des moyens de blocage de l'avancée de la tige. Enfin, l'étape de piqûre reste manuelle pour que l'auto-injecteur soit utilisable facilement par tous.

Selon des aspects avantageux mais non obligatoires de l'invention, un injecteur automatique peut incorporer une ou plusieurs des caractéristiques suivantes, prises en toute combinaison techniquement admissible :
- Le premier levier est élastiquement déformable et est déformé en début d'injection au contact de la came, pour basculer hors de la trajectoire de passage de la crémaillère.
- Le premier levier est configuré pour reprendre sa forme initiale par rappel élastique si l'embout revient vers sa position avancée en cours d'injection. Ce rappel élastique amène une partie du levier à se loger dans un cran de la crémaillère, empêchant ainsi l'avancée de la tige de poussée.
- Les ailettes comprennent chacune une surface d'appui sur une butée, qui est inclinée par rapport à un axe longitudinal de la tige parallèle à l'axe longitudinal et qui est complémentaire d'une surface de contact de la butée.
- L'injecteur automatique comprend, en outre, des moyens de verrouillage de l'embout dans une position avancée de fin d'injection, qui s'opposent au recul de l'embout après l'injection.
- Les moyens de verrouillage de l'embout comprennent un doigt élastique appartenant à la tige, qui pivote en fin d'injection par rappel élastique, pour venir bloquer le recul des ailettes de l'embout.
- Les moyens de verrouillage comprennent un deuxième levier de blocage du déplacement de l'embout, qui est prévu pour coopérer avec un pêne porté par l'embout, ce deuxième levier s'opposant au recul du pêne de l'embout lorsque l'embout est en position de fin d'injection.
- Le deuxième levier est élastiquement déformable et en ce que la tige comprend une enclume de maintien du deuxième levier pendant l'injection, qui empêche le deuxième levier de se déformer au contact du pêne tant que l'injection n'est pas terminée.
- L'enclume est agencée sur la tige pour cesser de maintenir le deuxième levier en fin d'injection, le deuxième levier se déformant alors au contact du pêne pour libérer le déplacement de l'embout vers sa position de fin d'injection et reprenant sa forme initiale par rappel élastique dans une position où il s'oppose au recul du pêne de l'embout.

L'invention et d'autres avantages de celle-ci apparaitront plus clairement à la lumière de la description qui va suivre de deux modes de réalisation d'un injecteur automatique conforme à l'invention, faite conformément à son principe et en référence aux dessins dans lesquels :
- la figure 1 est une vue en perspective éclatée d'un injecteur automatique conforme à l'invention,
- la figure 2 est une vue de côté de l'injecteur automatique de la figure 1 où l'injecteur est représenté en position assemblée et où un capot extérieur de l'injecteur est omis,
- la figure 3 est une coupe longitudinale de l'injecteur automatique des figures 1 et 2,
- les figures 4 à 11 représentent respectivement des vues en perspective de l'injecteur automatique des figures 1 à 3, dans lesquelles le capot extérieur de l'injecteur est omis et qui représentent des positions successives de l'injecteur durant l'injection, et
- les figures 12 à 16 sont des vues en perspectives, à différentes échelles, d'un injecteur automatique selon un deuxième mode de réalisation de l'invention, qui représentent des positions successives de l'injecteur durant l'injection.

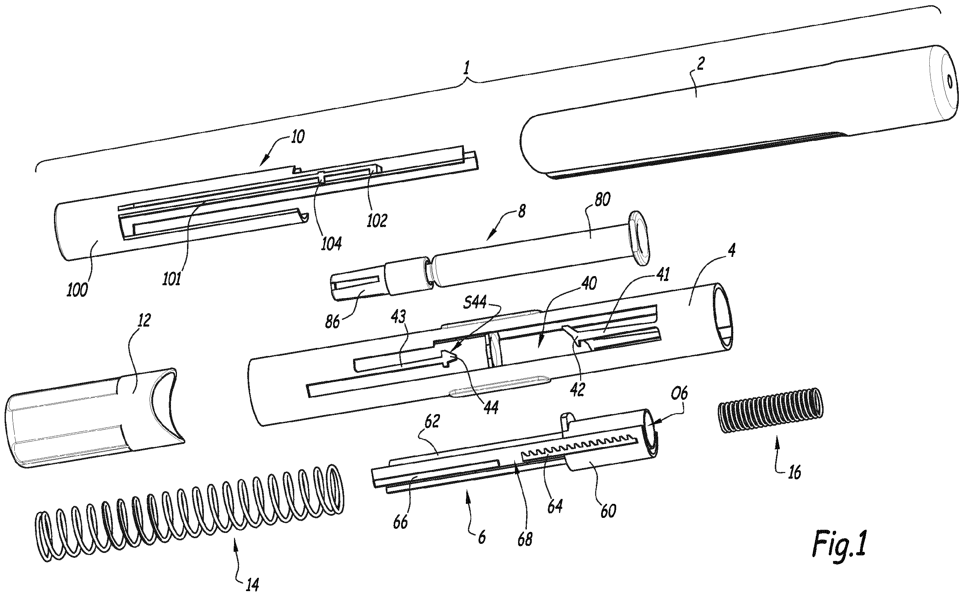

Sur la figure 1 est représenté un injecteur automatique ou auto-injecteur 1. A la figure 1, tous les composants de l'auto-injecteur 1 ne sont pas représentés sur une même ligne pour la clarté du dessin. Cependant, tous les composants de l'injecteur 1 sont en pratique alignés selon un même axe longitudinal X1. En effet, l'auto-injecteur 1 a l'allure d'un stylo, c'est-à-dire qu'il a une forme globalement cylindrique centrée sur l'axe X1.

L'injecteur 1 comprend une coque extérieure, ou capot 2 qui est globalement à géométrie tubulaire, centrée sur l'axe longitudinal X1 en configuration montée de l'injecteur 1.

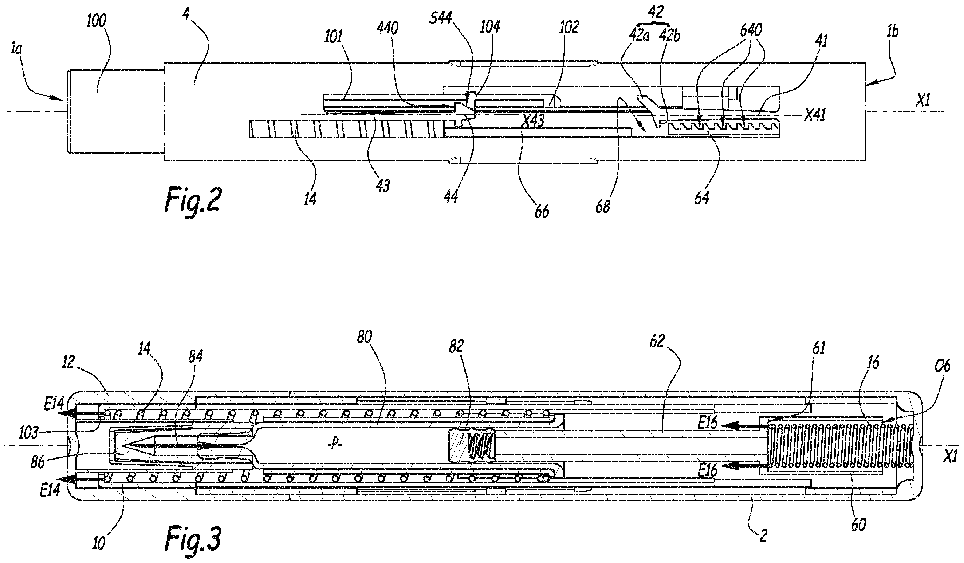

Comme visible à la figure 2, l'injecteur 1 comprend une première extrémité longitudinale 1a, qui est prévue pour être tournée vers l'épiderme lors d'une injection et une deuxième extrémité longitudinale 1b, qui est opposée à l'extrémité 1a le long de l'axe X1.

Aux figures 2 et 4 à 11, le capot 2 est omis pour une meilleure visualisation des composants internes de l'injecteur 1.

Une enveloppe tubulaire 4 est disposée coaxialement à l'intérieur du capot 2 et est immobile par rapport à celui-ci.

Dans la suite de la description, la direction « avant » désigne une direction allant de l'extrémité 1b vers l'extrémité 1a et inversement pour la direction « arrière ». De même, une direction longitudinale désigne une direction parallèle à l'axe longitudinal X1.

Un embout, ou poussoir, 10, de géométrie globalement tubulaire, est disposé coaxialement à l'intérieur de l'enveloppe 4. L'embout 10 comprend une partie avant 100 qui dépasse vers l'avant par rapport à l'enveloppe 4 lorsque l'injecteur 1 est au repos. L'embout 10 est soumis à l'action de charge élastique d'un ressort spiral 14, qui est agencé à l'intérieur de l'enveloppe tubulaire 4, au contact de l'embout 10.

La partie 100 comporte une extrémité avant 103 qui est radialement recourbée vers l'intérieur par rapport à l'axe X1. L'extrémité 103 forme un épaulement radial interne sur lequel le ressort spiral 14 est en appui, en exerçant un effort E14 de charge élastique dirigé vers l'avant. L'embout 10 comporte également une patte 101 qui s'étend longitudinalement à partir de la partie 100 vers l'arrière. Cette patte 101 porte un pêne 104 et une came 102 qui est disposée à l'arrière du pêne 104.

L'enveloppe 4 contient également une seringue 8 d'administration, ou d'injection d'un médicament P. En pratique, le médicament, ou principe actif P, peut être une solution d'atropine, qui est un antidote à une intoxication au gaz, ou une solution d'adrénaline, utilisée en cas de malaise cardiaque ou de crise allergique grave. La seringue 8 comporte un corps en verre 80, une aiguille creuse 84 et un piston 82 qui est mobile axialement à l'intérieur du corps 80. Le piston 82 est parfois appelé « joint de piston » dans le domaine médical puisqu'il s'agit d'un joint d'étanchéité en élastomère. L'aiguille 84 est collée au corps 80 et est protégée par un cache, ou protège-aiguille 86. Par ailleurs, la partie 100 de l'embout 10 est prévue pour protéger l'aiguille 84 lorsque le cache 86 est retiré, ceci afin d'éviter une piqûre accidentelle.

Une tige 6 de poussée du piston 82 à l'intérieur du corps de seringue 80 est également disposée à l'intérieur de l'enveloppe tubulaire 4. Cette tige 6 est en matière plastique et est agencée axialement avec la seringue 8. Elle est en plus soumise à l'action de charge d'élastique d'un ressort spiral 16.

Enfin, un capuchon 12 permet de boucher l'injecteur automatique 1 lorsque celui-ci n'est pas utilisé. Le capuchon 12 est solidaire axialement, ou en translation, avec le protège-aiguille 86. En effet, le capuchon 12 comprend des pattes qui entourent le protège-aiguille 86 et qui sont attachées, ou « clipsées » dans le protège-aiguille 86. Ainsi, le retrait du capuchon 12 entraine solidairement le retrait du protège-aiguille 86 de la seringue 8.

L'enveloppe tubulaire 4 délimite une découpe 40, dont le contour est globalement rectangulaire. Un levier d'injection 41 s'étend, à partir d'un bord latéral arrière de la découpe 40, vers l'avant. X41 désigne un axe longitudinal du levier 41. Le levier 41 comporte une extrémité avant 42, formée de deux branches 42a et 42b qui s'étendent transversalement à l'axe longitudinal X41 du levier 41.

Par ailleurs, un levier de garde 43 s'étend vers l'arrière à partir d'un bord avant de la découpe 40, c'est-à-dire à partir d'un bord opposé au bord d'attache du levier 41. X43 désigne un axe longitudinal du levier 43. Ce levier de garde 43 comporte une extrémité arrière 44, ayant une surface S44 de contact avec le pêne 104 et un épaulement 440 de blocage du pêne 104. La surface S44 est une surface inclinée, qui converge vers l'arrière en direction de l'axe X43. L'enveloppe 4 est réalisée en matière plastique et les leviers 41 et 43 sont élastiquement déformables.

La tige 6 de poussée du piston 82 comporte, à l'arrière, un corps tubulaire 60 comportant une ouverture O6 de passage du ressort 16 et une queue creuse 62 qui s'étend à partir du corps 60 vers l'avant et qui est prévue pour pousser le piston 82 durant l'injection. A cet effet, la queue 62 est partiellement insérée dans le corps de seringue 80, au contact du piston 82. La tige 6 comprend un épaulement radial interne 61, qui est disposé à la jonction entre le corps 60 et la queue 62 et qui forme une surface d'appui pour le ressort spiral 16. Le ressort spiral 16 exerce un effort E16 de charge élastique dirigé vers l'avant sur l'épaulement 61.

La tige 6 porte une crémaillère longitudinale 64 pourvue de plusieurs crans 640. Cette crémaillère 64 est disposée à l'arrière de la queue 62, notamment au même niveau axial que le corps 60. La tige 6 porte également une enclume 66, qui est disposée à l'avant par rapport à la crémaillère 64. L'enclume 66 et la crémaillère 64 définissent entre elles un espace creux, ou logement 68.

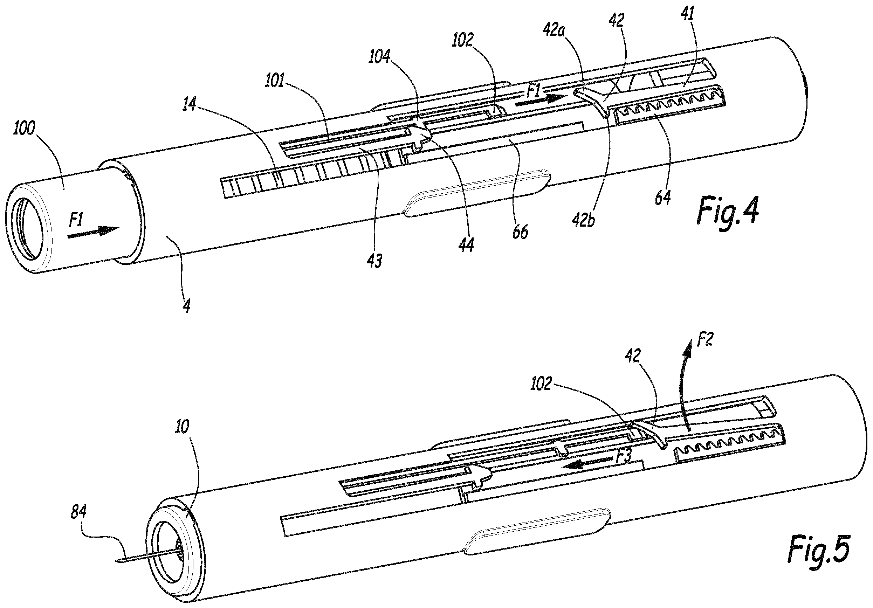

L'embout 10 est mobile axialement, autour de l'aiguille 84 et coaxialement à l'intérieur de l'enveloppe 4, entre une position avancée, où il protège, ou entoure, l'aiguille 84 et une position reculée, où l'aiguille 84 est découverte. La position reculée est, par exemple, représentée à la figure 5. Dans l'exemple, la position avancée est une position armée de l'auto-injecteur 1, qui est représentée à la figure 4. Dans cette position, l'auto-injecteur 1 est prêt à l'emploi, c'est-à-dire que le cache 86 est retiré.

Le déplacement de l'embout de sa position avancée à sa position reculée s'effectue à l'encontre de l'effort E14 de charge élastique du ressort spiral 14, c'est-à-dire que le ressort 14 se comprime lors du recul de l'embout 10.

En position avancée de l'embout 10, le levier d'injection 41 bloque l'avancée de la tige 6. En effet, la branche 42b de l'extrémité 42 appartenant au levier 41 bloque l'avancée de la crémaillère 64 équipant la tige 6. Plus précisément, la branche 42b de l'extrémité 42 est disposée sur la trajectoire d'avancée de la crémaillère 64. Autrement dit, la branche 42b forme un obstacle au passage de la crémaillère 64.

Le levier 41 et la crémaillère 64 forment donc ensemble des moyens de blocage de l'avancée de la tige 6. Ces moyens de blocage de l'avancée de la tige 6 sont initialement activés lorsque l'embout 10 se trouve en position avancée.

Dans la position avancée de l'embout 10, le pêne 104 de la patte 101 bute contre la surface S44 de l'extrémité 44 du levier 43. Plus précisément, le pêne 104 est en appui contre la surface S44 vers l'avant. L'extrémité 44 du levier 43 bloque donc l'avancée du pêne 104 et la surface S44 est une surface de blocage de l'avancée de l'embout 10.

Plus précisément, l'extrémité 44 du levier 43 est intercalée, selon une direction orthoradiale à l'axe X1, entre le pêne 104 et l'enclume 66 de la tige 6. En effet, l'extrémité 44, le pêne 104 et l'enclume 106 sont disposés au même niveau axial et l'extrémité 44 est coincée, en considérant une direction orthoradiale à l'axe X1, entre le pêne 104 et l'enclume 66. L'enclume 66 empêche ainsi le levier 43 de se déformer élastiquement sous l'action d'appui du pêne 104, cette action étant dirigée vers l'avant. Le levier 43 conserve donc sa forme initiale et empêche le pêne 104 de se déplacer vers l'avant, sous l'action du ressort 14.

Pour réaliser une injection, l'utilisateur retire le capuchon 12 de l'injecteur 1 et emmène avec le capuchon 12 le protège-aiguille 86 puisque ceux-ci sont solidaires axialement. L'injecteur est alors prêt à l'emploi et l'embout 10 se trouve dans sa position avancée, comme représenté à la figure 4.

Ensuite, l'utilisateur appuie l'injecteur 1 sur une partie de son corps, comme sa cuisse. Pour ce faire, l'utilisateur amène l'extrémité 1a de l'injecteur 1 contre sa cuisse et appuie, par exemple, sur l'extrémité 1b en direction de sa cuisse. Cela entraîne un recul de l'embout 10 au contact de la peau, ce recul étant représenté par la flèche F1 à la figure 4. L'embout 10 découvre donc l'aiguille 84 dans son déplacement et l'aiguille 84 pénètre dans l'épiderme en parallèle du recul F1 de l'embout 10. La came 102 de la patte 101 recule avec le reste de l'embout 10 et vient alors au contact de l'extrémité 42 du levier d'injection 41, c'est-à-dire que l'embout 10 parvient en position reculée.

Comme visible à la figure 5, lorsque la came 102 parvient au contact de l'extrémité 42 du levier 41, le levier 41 se déforme élastiquement sous l'action de poussée de la came 102 vers l'arrière. Cette déformation du levier 41 est une flexion F2 qui est dirigée de manière opposée à la crémaillère 64, selon une direction globalement orthoradiale à l'axe X1. La flexion F2 entraine que la branche 42b de l'extrémité 42 se dégage de la trajectoire d'avancée de la crémaillère 64 c'est-à-dire que le levier 41 bascule vers une configuration dégagée de la crémaillère 64, ce qui libère le mouvement avancée de la tige 6 sous l'effort E16 de charge élastique du ressort 16. La tige peut alors se déplacer vers l'avant, comme représenté par la flèche F3 à la figure 5. Ainsi, le déplacement de l'embout 10 dans sa position reculée entraine la désactivation des moyens de blocage de l'avancée de la tige 6.

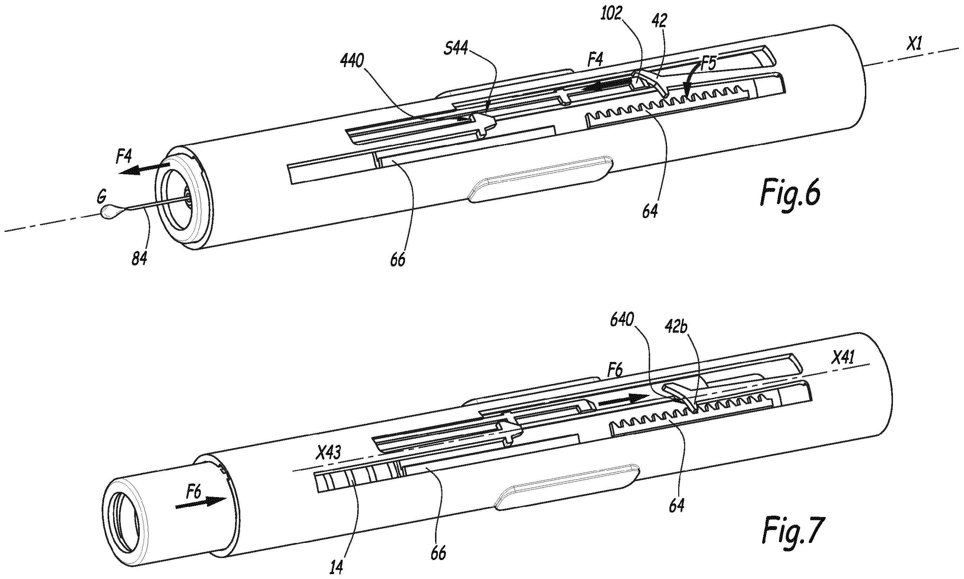

Le déplacement vers l'avant F3 de la tige 6 entraine le démarrage de l'injection, c'est-à-dire que le piston 82 est poussé à l'intérieur du corps de seringue 80 par la queue 62 et force le principe actif P contenu dans le corps 80 à sortir par l'aiguille creuse 84. L'injection du principe actif P de la seringue 8 est représentée à la figure 6 par l'apparition d'une goutte G à l'extrémité de l'aiguille 84.

Par ailleurs, si l'utilisateur relâche de manière partielle la pression de l'injecteur 1 sur sa cuisse, l'embout 10 quitte sa position reculée, c'est-à-dire qu'il revient vers sa position avancée grâce à l'effort E14, par détente, ou retour élastique, du ressort 14. Ainsi, la came 102 cesse d'agir sur le levier d'injection 41 et la branche 42b revient élastiquement en prise avec la crémaillère 64. Le déplacement, en cours d'injection, de l'embout 10 vers sa position avancée entraine donc la réactivation des moyens de blocage de l'avancée de la tige 6. Dans ce mode de réalisation, il n'est pas nécessaire que l'embout 10 parvienne en position avancée pour désactiver les moyens de blocage de l'avancée de la tige 6. En effet, dès lors que l'embout 10 quitte sa position reculée, c'est-à-dire que l'utilisateur relâche la pression de l'auto-injecteur 1 sur sa cuisse, les moyens de blocage de l'avancée de la tige 6 sont réactivés.

Dans le cas où l'utilisateur retire complètement l'auto-injecteur 1 de sa cuisse, l'embout 10 parvient dans sa position reculée et est bloqué dans son déplacement vers l'avant. En effet, comme expliqué plus haut, le mouvement du pêne 104 vers l'avant est bloqué par l'extrémité 44 du levier 43, en coopération avec l'enclume 66.

Le relâchement partiel ou total de la pression de l'auto-injecteur 1 sur le corps de l'utilisateur entraine un retour de l'embout 10 vers sa position avancée, comme représenté par des flèches F4 à la figure 6. Suite à ce déplacement, le levier 41 reprend sa forme initiale par retour élastique en direction de la crémaillère 64. Le mouvement de retour élastique du levier 41 est représenté à la figure 6 par une flèche F5 et implique que la branche 42b de l'extrémité 42 vient se loger dans un cran 640 de la crémaillère 64. La crémaillère 64 est alors bloquée dans son mouvement par le levier 41. La tige 6 est alors immobilisée et l'injection du médicament est provisoirement stoppée.

Ainsi, si l'utilisateur retire, pour une raison quelconque l'injecteur 1 de sa cuisse, l'injection est stoppée et il n'y a pas de perte de médicament. Cela est particulièrement avantageux puisque, dans les situations d'urgence comme un malaise cardiaque, une crise d'allergie ou encore une attaque au gaz chimique, l'utilisateur est généralement en état de stress et peut être amené à trembler et donc, à retirer accidentellement l'auto-injecteur de sa cuisse.

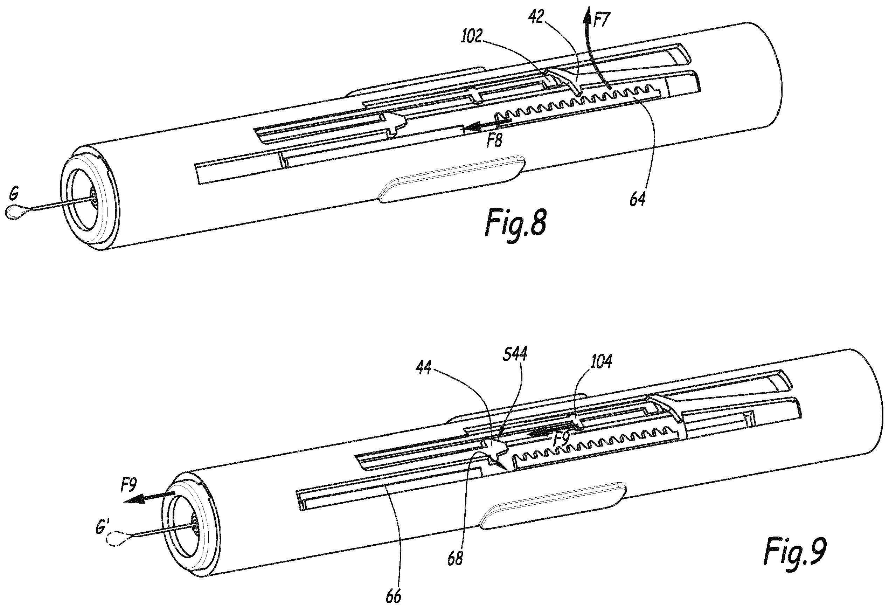

L'utilisateur peut par la suite reprendre l'injection en appuyant à nouveau sur sa cuisse pour faire reculer l'embout 10. Comme décrit précédemment, un nouveau recul de l'embout 10, représenté par la flèche F6 à la figure 7, entraine un recul sur la même course de la came 102 qui revient au contact de l'extrémité 42 du levier d'injection 41. L'action de contact de la came 102 au contact de l'extrémité 42 entraine à, nouveau un basculement F7 du levier 41, hors des crans 640 de la crémaillère 64. La crémaillère 64 est alors libérée de l'action de retenue du levier 41 et le mouvement d'avancée de la tige 6 se poursuit, comme représenté par la flèche F8 à la figure 8. Cela signifie que l'injection continue, comme représenté par la goutte G à la figure 8.

Lorsque l'injection est terminée, généralement après une durée de 4 à 8 secondes, l'utilisateur retire l'auto-injecteur 1 de sa cuisse et l'embout 10 avance sous l'effet de l'effort E14, comme représenté par les flèches F9 à la figure 9. A la figure 9, la fin de la phase d'injection est représentée par une goutte G' dont le contour est schématiquement dessiné en traits interrompus.

En fin d'injection, l'extrémité 44 du levier 43 n'est plus disposée au même niveau axial que l'enclume 66 puisque l'enclume 66 s'est déplacée vers l'avant suite à l'avancée de la tige 6 et a dépassé l'extrémité 44. L'enclume 66 se trouve alors devant l'extrémité 44 du levier 43. Plus précisément, l'extrémité 44 se trouve au même niveau axial que le logement 68 interposé entre l'enclume 66 et la crémaillère 64.

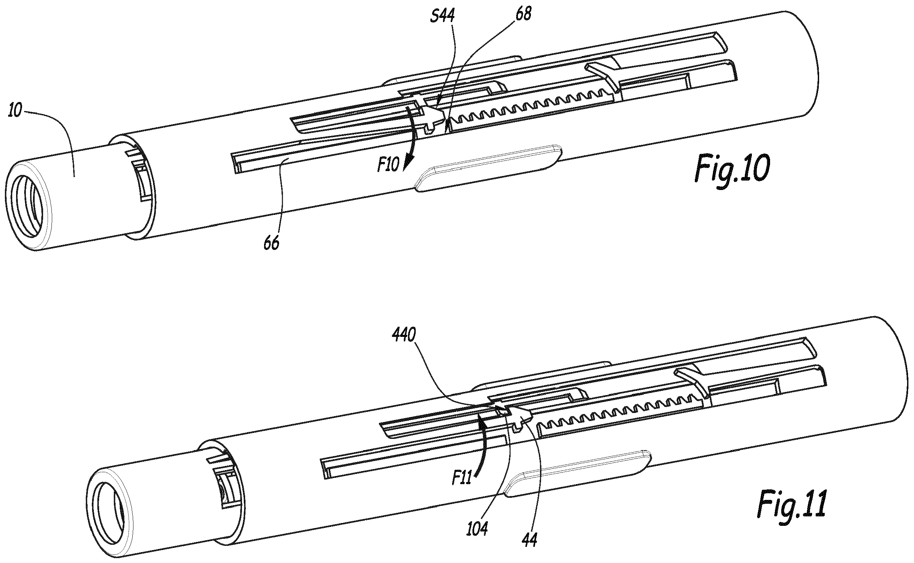

Lors de l'avancée de l'embout 10, le pêne 104 vient au contact de la surface S44 de l'extrémité 44 et l'effort de poussée du pêne 102 vers l'avant, sur la surface S44, entraine un basculement F10 du levier 43 de manière que l'extrémité 44 vienne se loger temporairement dans le logement 68 de la tige 6. En effet, l'enclume 66 n'empêche plus la déformation élastique du levier de garde 43. Le pêne 104 de l'embout 10 passe donc devant l'extrémité 44 du levier 43. L'embout 10 se trouve alors dans une position avancée de fin d'injection représentée à la figure 11, qui est une position encore plus en avant par rapport à la position avancée.

Dès que le pêne 104 a dépassé l'extrémité 44, c'est-à-dire lorsque l'embout 10 parvient en position de fin d'injection, le levier 43 reprend sa forme initiale par retour élastique F11. Dès lors, l'épaulement 440 de l'extrémité 44 s'oppose au recul de l'embout 10. En d'autres termes, l'épaulement 440 forme une butée de blocage du mouvement de recul du pêne 104. Le levier 43 et le pêne 104 forment donc ensemble des moyens de verrouillage de l'embout 10 en position avancée de fin d'injection, qui s'opposent au recul de l'embout après l'injection. Cela constitue une sécurité pour l'utilisateur puisque l'aiguille 84 ne peut plus être découverte après que l'injecteur est été utilisé. Il n'y a donc pas de risque d'une piqure accidentelle par l'aiguille 84 et l'embout 10 est maintenu en position de fin d'injection.

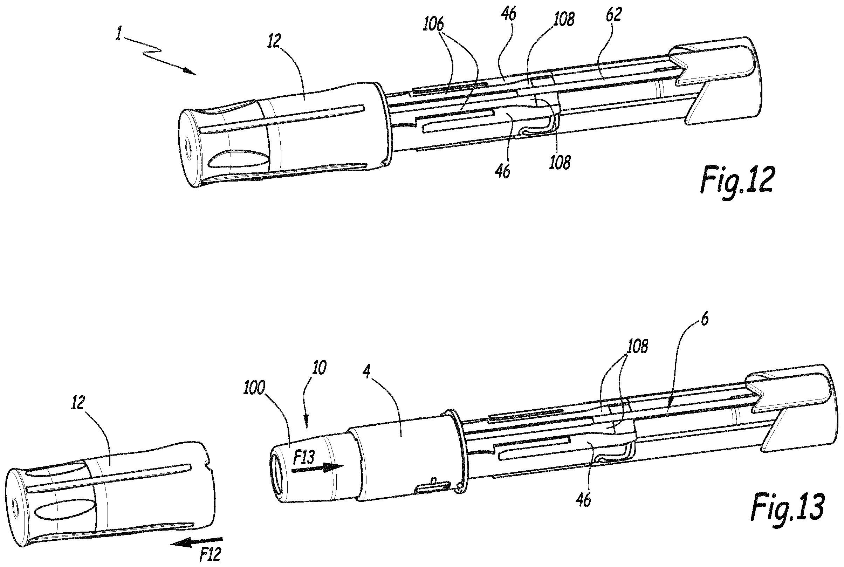

Sur les figures 12 à 16 est représenté un deuxième mode de réalisation de l'injecteur automatique 1. Dans ce qui suit, seules les différences par rapport au premier mode de réalisation sont décrites dans un souci de concision. De plus, les éléments de l'injecteur 1 qui sont identiques ou qui fonctionnent de manière semblable à ceux de l'injecteur du premier mode de réalisation conservent leur référence numérique, alors que les éléments additionnels ou qui fonctionnent différemment portent d'autres références numériques.

Dans ce deuxième mode de réalisation, l'embout 10 comporte également une partie avant 100 qui dépasse de l'enveloppe tubulaire 4 vers l'avant. Deux ailettes 106 s'étendent à partir du corps 100 vers l'arrière. Ces ailettes 106 sont élastiquement déformables et comportent chacune une extrémité arrière 108. Par ailleurs, l'auto-injecteur 1 est symétrique par rapport à un plan médian contenant l'axe longitudinal X1. Ainsi, deux autres ailettes sont disposées du côté opposé à l'angle de vue des figures 12 à 16. Dans la suite de la description, seuls les éléments disposés du côté visible sont décrits car les éléments symétriques correspondants sont identiques.

Une queue 62 appartenant à une tige 6 de poussée du piston à l'intérieur du corps de seringue est prise en étau entre les deux extrémités 108 des ailettes 106. Plus précisément, la queue 62 présente une largeur, mesurée selon une direction orthoradiale à l'axe X1, qui est supérieure ou égale à l'écartement entre les ailettes 106, mesuré selon la même direction.

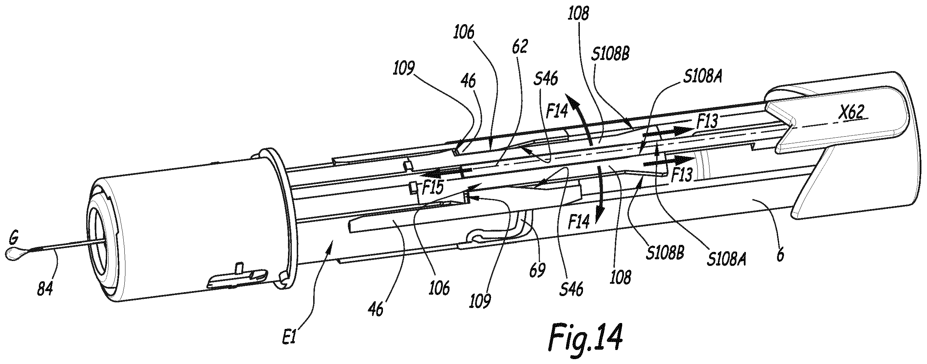

Les extrémités 108 des ailettes 106 comportent chacune une surface S108A d'appui contre la queue 62 de la tige 6 et une surface inclinée S108B, qui est opposée à la surface d'appui S108A sur la queue 62. Plus précisément, les surfaces S108B divergent, par rapport à un axe longitudinal X62 de la queue 62, vers l'arrière. Les surfaces S108A et S108B sont repérées à la figure 14 où seule la trace radiale externe des surfaces S108A est visible puisque celles-ci sont en appui contre la queue 62. Ces surfaces S108A sont parallèles entre elles et à l'axe X1.

Les surfaces S108B sont complémentaires de deux surfaces S46 appartenant à deux sièges 46. Autrement formulé, les surfaces S46 divergent vers l'arrière par rapport à l'axe longitudinal X62 de la queue 62, avec la même inclinaison que celle des surfaces S108B. Les sièges 46 appartiennent à l'enveloppe 4 et sont disposés à un même niveau axial. Les ailettes 106 sont disposées entre ces deux sièges 46 et de part et d'autre de la queue 62, par rapport à une direction orthoradiale à l'axe X1.

La tige 6 est soumise en permanence à un effort de charge élastique d'un ressort 16, qui tend à pousser la tige 6 vers l'avant. Cet effort de poussée de la tige 6, qui est analogue à l'effort E16 du premier mode de réalisation, tend à écarter les ailettes 106 l'une de l'autre par déformation élastique, pour laisser passer la queue 62.

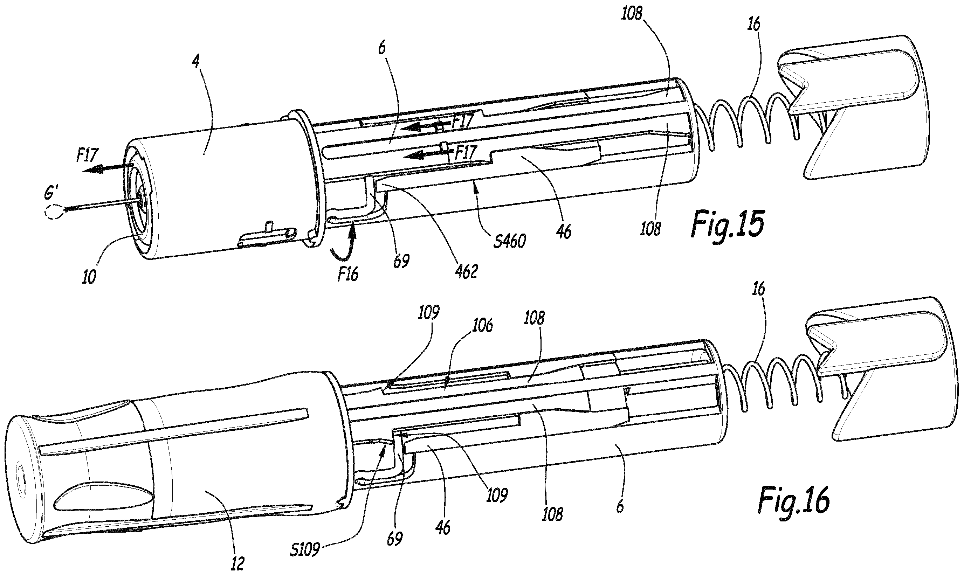

Comme dans le premier mode de réalisation, l'embout 10 est mobile axialement à l'encontre de l'action de charge élastique d'un ressort non représenté et comparable au ressort 14, entre une position avancée, représentée à la figure 13 et une position reculée, représentée à la figure 15.

Au repos, c'est-à-dire en position avancée de l'embout 10, qui est représentée aux figures 12 et 13, les surfaces S108B des extrémités 108 des ailettes 106 sont en appui contre les surfaces S46 des sièges 46. Ainsi, les sièges 46 empêchent les ailettes 106 de se déformer élastiquement pour laisser passer la queue 62 de la tige 6. Les sièges 46 forment donc, avec les ailettes 106, des moyens de blocage de l'avancée de la tige 6. Ces moyens de blocage de l'avancée de la tige 6 sont initialement activés lorsque l'embout 10 se trouve en position avancée.

Par ailleurs, un doigt 69 porté par la tige de poussée 6 est en contact avec une surface S460 d'un siège 46, du côté opposé aux ailettes 106. Ce doigt 69 est un doigt élastique, ou élastiquement déformable. Au repos et plus généralement tant que l'injection n'est pas terminée, le doigt 69 est écrasé, ou comprimé contre la surface S460 des butées 46. Par ailleurs, la surface S460 est opposée à la surface S46 des butées 46.

Les ailettes 106 comportent par ailleurs, à l'avant par rapport aux extrémités 108, chacune un épaulement 109 qui élargit les ailettes 106 et qui est tourné vers l'arrière. S109 désigne une surface latérale des ailettes 106, cette surface étant disposée dans le prolongement de la surface S108 vers l'avant, après avoir passé l'épaulement 109.

Pour réaliser une injection, l'utilisateur retire le capuchon 12 comme représenté par la flèche F12 à la figure 13 et emmène, comme dans le premier mode de réalisation, le protège-aiguille 86 avec le capuchon 12 puisque ceux-ci sont solidaires axialement.. Puis, l'utilisateur applique l'injecteur 1 sur une partie de son corps, comme sa cuisse. L'action de contact de l'injecteur 1 sur la cuisse de l'utilisateur tend à faire reculer l'embout 10, comme représenté par la flèche F13 à la figure 13. Les extrémités 108 des ailettes 106 décollent alors des sièges 46, comme illustré à la figure 14.

Dans cette position, les extrémités 108 des ailettes 106 ne sont plus maintenues par les sièges 46 en appui ferme contre la queue 62, si bien que les ailettes 106 peuvent se déformer élastiquement. L'action de charge élastique du ressort 16 entraine alors la déformation élastique des ailettes 106 pour laisser passer la queue 62 de la tige 6. Plus précisément, les ailettes 106 s'écartent l'une de l'autre, ou fléchissent, pour laisser passer la queue 62, comme représenté par les flèches F14 à la figure 14. Le fléchissement F14 des ailettes 106 s'effectue en pratique transversalement à l'axe X62. Ainsi, la tige 6 coulisse axialement vers l'avant et le piston est déplacé à l'intérieur du corps de seringue. Par conséquent, lorsque l'embout 10 quitte sa position avancée, c'est-à-dire se déplace vers sa position reculée, les moyens de blocage de l'avancée de la tige 6 sont désactivés et l'injection commence, comme symbolisé par une goutte G à la figure 14. L'avancée de la tige 6 est représentée à la figure 14 par une flèche F15. Dans ce mode de réalisation, il n'est pas nécessaire que l'embout 10 parvienne en position reculée pour désactiver les moyens de blocage de l'avancée de la tige 6. En effet, ces moyens sont désactivés dès lors que l'embout 10 quitte sa position avancée. Par ailleurs, le doigt élastique 69 frotte contre la surface S460 de la butée 46 pendant le déplacement de la tige 6.

Si l'utilisateur retire accidentellement l'injecteur 1 de sa cuisse pendant l'injection, l'embout 10 revient dans sa position avancée sous l'action de charge élastique du ressort comprimé lors du recul de l'embout 10.

Lorsque l'embout 10 parvient en position avancée, les surfaces S108B des extrémités 108 des ailettes 106 arrivent en butée contre les surfaces S46 des sièges 46. Les ailettes 106 reprennent leur position initiale au contact des sièges 46 et les sièges 46 empêchent alors de nouveau la déformation élastique des ailettes 106. La queue 62 de la tige 6 est de nouveau maintenue en étau entre les deux ailettes 106, ce qui empêche l'avancée de la tige 6. Ainsi, le retour de l'embout 10 dans sa position avancée entraine la réactivation des moyens de blocage de l'avancée de la tige 6 et l'injection est alors provisoirement stoppée. Dans ce mode de réalisation, il est nécessaire que l'embout 10 parvienne en position avancée pour profiter de l'appui des sièges 46 et ainsi réactiver les moyens de blocage de l'avancée de la figure 6.

Pour continuer l'injection, l'utilisateur applique de nouveau l'injecteur 1 sur sa cuisse et appuie pour faire reculer l'embout 10. Comme expliqué précédemment, le recul de l'embout 10 implique la reprise du mouvement de l'avancée de la tige 6 et la poursuite de l'injection.

En fin d'injection, le doigt 69 de la tige 6 n'est plus bloqué par le siège 46 et reprend sa forme initiale par retour élastique, comme représenté par la flèche F16 à la figure 15. Le rappel élastique du doigt 69 pour être interprété comme un mouvement de pivotement et le doigt 69 vient se loger dans un espace E1 disposé entre une extrémité avant 462 du siège 46 et l'épaulement 109 d'une ailette 106. A la figure 15, la fin de la phase d'injection est symbolisé par une goutte G' dont le contour est réalisé en traits interrompus.

Lorsque l'utilisateur retire l'auto-injecteur 1 de sa cuisse, l'embout 10 avance sous l'action de charge élastique du ressort et revient en position avancée. Le doigt 69 de la tige 6 est de nouveau légèrement comprimé au contact de la surface S109 de l'ailette 106, pour laisser passer les ailettes 106. Autrement dit, les ailettes 106 forcent le passage vers la position avancée en comprimant le doigt 69. Ensuite, dès que l'épaulement 109 à dépassé le doigt 69, c'est-à-dire que l'épaulement 109 est parvenu devant le doigt 69, le doigt 69 reprend sa forme initiale par retour élastique et vient se loger contre l'épaulement 109 de l'ailette 106. Ainsi, le doigt 69 forme un obstacle au recul des ailettes 106 de l'embout 10. Le doigt 69 peut donc être interprété comme un moyen de verrouillage de l'embout 10 dans une position avancée de fin d'injection. Ce moyen s'oppose au recul de l'embout 10 après l'injection. Ainsi, l'utilisateur ne risque pas de se blesser avec l'aiguille 84 en appuyant accidentellement sur l'embout 10 puisque l'embout 10 est bloqué en position avancée.

En variante, l'injecteur automatique 1 peut être utilisé pour effectuer une piqure sur tout autre partie du corps que la cuisse.

En variante, le principe actif P utilisé peut être un médicament autre qu'une solution d'adrénaline ou d'atropine.

En variante non représentée, l'injecteur automatique 1 peut être rechargeable.

Les caractéristiques techniques des variantes et modes de réalisation envisagés ci-dessus peuvent être combinées entre elles pour donner de nouveaux modes de réalisation de l'invention.

## Revendications

1. Injecteur automatique (1), comprenant :
- un capot (2), s'étendant selon un axe longitudinal (X1),
- une seringue (8) d'injection d'un médicament (P), qui est disposée à l'intérieur du capot et qui comporte une aiguille (84), un protège-aiguille (86), un corps (80) et un piston (82),
- une tige (6) de poussée du piston à l'intérieur du corps de seringue, qui est déplacée axialement vers l'avant par un effort (E16) de charge élastique exercé par un premier ressort (16), lors d'une injection,
- un embout (10) de protection de l'aiguille, qui est déplaçable axialement autour de l'aiguille et à l'encontre d'un effort (E14) de charge élastique exercé par un deuxième ressort (14), entre une position avancée (figure 2 ; figure 13) où il entoure l'aiguille et une position reculée (figure 8 ; figure 14), où l'aiguille est découverte,
- des moyens (41, 64 ; 106, 46) de blocage de l'avancée de la tige,
**caractérisé en ce que** :
- les moyens de blocage (41, 64 ; 106, 46) sont configurés pour être désactivés lorsque l'embout (10) est déplacé vers sa position reculée (figure 8 ; figure 14) ou parvient dans cette position, et pour être réactivés en cours d'injection lorsque l'embout est déplacé vers sa position avancée (figure 2 ; figure 13) ou parvient dans cette position, et
- les moyens (41, 64) de blocage de l'avancée de la tige (6) comprennent :
- un premier levier (41) de blocage du déplacement de la tige, qui est prévu pour bloquer l'avancée d'une crémaillère (64) ménagée sur la tige de poussée (6), l'embout (10) comprenant une came (102) de libération de l'action de blocage du premier levier (41) sur la crémaillère (64), cette came entrainant le basculement (F2) du premier levier vers une configuration dégagée de la crémaillère lorsque l'embout parvient en position reculée, ou
- deux ailettes élastiques (106), qui appartiennent à l'embout (10) et qui sont disposées de part et d'autre de la tige de poussée (6), et deux butées (46) de bocage des ailettes élastiques dans une position (figure 13) où les ailettes élastiques maintiennent entre elles la tige en étau.

2. Injecteur automatique selon la revendication 1, **caractérisé en ce que** le premier levier (41) est élastiquement déformable et est déformé en début d'injection au contact de la came (102), pour basculer hors de la trajectoire de passage de la crémaillère (64).

3. Injecteur automatique selon la revendication 2, **caractérisé en ce que** le premier levier (41) est configuré pour reprendre sa forme initiale par rappel élastique (F5) si l'embout revient vers sa position avancée en cours d'injection et **en ce que** ce rappel élastique (F5) amène une partie (42b) du levier à se loger dans un cran (640) de la crémaillère (64), empêchant ainsi l'avancée de la tige de poussée (6).

4. Injecteur automatique selon la revendication 1, **caractérisé en ce que** les ailettes (106) comprennent chacune une surface (S108B) d'appui sur une butée (46), qui est inclinée par rapport à un axe (X62) longitudinal de la tige (6) parallèle à l'axe longitudinal (X1) et qui est complémentaire d'une surface de contact (S46) de la butée.

5. Injecteur automatique selon l'une des revendications précédentes, **caractérisé en ce qu'**il comprend, en outre, des moyens (43, 104 ; 69) de verrouillage de l'embout (10) dans une position avancée de fin d'injection (figure 11 ; figure 16), qui s'opposent au recul de l'embout après l'injection.

6. Injecteur automatique selon la revendication 5, **caractérisé en ce que** les moyens de verrouillage de l'embout comprennent un doigt élastique (69) appartenant à la tige (6), qui pivote (F16) en fin d'injection par rappel élastique pour venir bloquer le recul des ailettes (106) de l'embout (10).

7. Injecteur automatique selon la revendication 5, **caractérisé en ce que** les moyens de verrouillage comprennent un deuxième levier (43) de blocage du déplacement de l'embout (10), qui est prévu pour coopérer avec un pêne (104) porté par l'embout, ce deuxième levier s'opposant au recul du pêne de l'embout lorsque l'embout est en position de fin d'injection (figure 11).

8. Injecteur automatique selon la revendication 7, **caractérisé en ce que** le deuxième levier (43) est élastiquement déformable et **en ce que** la tige (6) comprend une enclume (66) de maintien du deuxième levier (43) pendant l'injection, qui empêche le deuxième levier de se déformer au contact du pêne tant que l'injection n'est pas terminée.

9. Injecteur automatique selon la revendication 8, **caractérisé en ce que** l'enclume (66) est agencée sur la tige (6) pour cesser de maintenir le deuxième levier (43) en fin d'injection, le deuxième levier se déformant alors au contact du pêne pour libérer le déplacement de l'embout vers sa position de fin d'injection et reprenant sa forme initiale par rappel élastique (F11) dans une position (figure 11) où il s'oppose au recul du pêne de l'embout.

## Patentansprüche

1. Automatischer Injektor (1), Folgendes aufweisend:
- eine Kappe (2), die sich entlang einer Längsachse (X1) erstreckt,
- eine Spritze (8) zum Injizieren eines Medikaments (P), die im Inneren der Kappe angeordnet ist und eine Nadel (84), einen Nadelschutz (86), einen Körper (80) und einen Kolben (82) hat,
- einen Schaft (6) zum Schieben des Kolbens im Inneren des Spritzenkörpers, der bei einer Injektion durch eine von einer ersten Feder (16) ausgeübte elastische Belastungskraft (E16) axial nach vorne verschoben wird,
- einen Stutzen (10) zum Schutz der Nadel, der axial um die Nadel herum und entgegen einer von einer zweiten Feder (14) ausgeübten elastischen Belastungskraft (E14) zwischen einer ausgefahrenen Position (Figur 2; Figur 13), in der er die Nadel umgibt, und einer eingefahrenen Position (Figur 8; Figur 14) verschiebbar ist, in der die Nadel offenliegt,
- Einrichtungen (41, 64; 106, 46) zum Blockieren des Ausfahrens des Schafts,
**dadurch gekennzeichnet, dass**:
- die Blockierungseinrichtungen (41, 64; 106, 46) dazu ausgelegt sind, desaktiviert zu sein, wenn der Stutzen (10) zu seiner eingefahrenen Position (Figur 8; Figur 14) verschoben wird oder diese Position erreicht, und im Verlauf einer Injektion reaktiviert zu sein, wenn der Stutzen zu seiner ausgefahrenen Position (Figur 2; Figur 13) verschoben wird oder diese Position erreicht, und
- die Einrichtungen (41, 64) zum Blockieren des Ausfahrens des Schafts (6) aufweisen:
- einen ersten Hebel (41) zum Blockieren der Verschiebung des Schafts, der vorgesehen ist, um die Vorwärtsbewegung einer Zahnstange (64) zu blockieren, die am Schiebeschaft (6) ausgebildet ist, wobei der Stutzen (10) einen Nocken (102) zur Freisetzung der Blockierungswirkung des ersten Hebels (41) an der Zahnstange (64) aufweist, wobei dieser Nocken das Kippen (F2) des ersten Hebels in eine von der Zahnstange freigesetzte Auslegung bewirkt, wenn der Stutzen die eingefahrene Position erreicht, oder
- zwei elastische Rippen (106), die zum Stutzen (10) gehören und beidseits des Schiebeschafts (6) angeordnet sind, und zwei Anschläge (46) zum Blockieren der elastischen Rippen in einer Position (Figur 13), in der die elastischen Rippen den Schaft zwischen sich eingespannt halten.

2. Automatischer Injektor nach Anspruch 1, **dadurch gekennzeichnet, dass** der erste Hebel (41) elastisch verformbar ist und zu Injektionsbeginn bei Kontakt des Nockens (102) verformt wird, um aus der Durchtrittsbahn der Zahnstange (64) zu kippen.

3. Automatischer Injektor nach Anspruch 2, **dadurch gekennzeichnet, dass** der erste Hebel (41) dazu ausgelegt ist, seine Ausgangsform durch elastische Rückstellung (F5) wieder anzunehmen, wenn der Stutzen im Verlauf einer Injektion in seine ausgefahrene Position zurückkehrt, und dass diese elastische Rückstellung (F5) einen Teil (42b) des Hebels dazu bringt, sich in eine Kerbe (640) der Zahnstange (64) einzupassen, wodurch das Ausfahren des Schiebeschafts (6) verhindert wird.

4. Automatischer Injektor nach Anspruch 1, **dadurch gekennzeichnet, dass** die Rippen (106) jeweils eine Fläche (S108B) zur Anlage an einem Anschlag (46) aufweisen, die in Bezug auf eine Längsachse (X62) des Schafts (6) parallel zur Längsachse (X1) geneigt und komplementär zu einer Kontaktfläche (S46) des Anschlags ist.

5. Automatischer Injektor nach einem der vorhergehenden Ansprüche, **dadurch gekennzeichnet, dass** er darüber hinaus Einrichtungen (43, 104; 69) zur Verriegelung des Stutzens (10) in einer ausgefahrenen Injektionsendposition (Figur 11; Figur 16) aufweist, die sich dem Rückzug des Stutzens nach der Injektion entgegensetzen.

6. Automatischer Injektor nach Anspruch 5, **dadurch gekennzeichnet, dass** die Einrichtungen zur Verriegelung des Stutzens einen zum Schaft (6) gehörenden elastischen Finger (69) aufweisen, der sich am Ende einer Injektion durch elastische Rückstellung verschwenkt (F16), um den Rückzug der Rippen (106) des Stutzens (10) zu blockieren.

7. Automatischer Injektor nach Anspruch 5, **dadurch gekennzeichnet, dass** die Verriegelungseinrichtungen einen zweiten Hebel (43) zum Blockieren der Verschiebung des Stutzens (10) aufweisen, der dazu vorgesehen ist, mit einem vom Stutzen getragenen Riegel (104) zusammenzuwirken, wobei sich dieser zweite Hebel dem Rückzug des Riegels des Stutzens entgegensetzt, wenn sich der Stutzen in der Injektionsendposition (Figur 11) befindet.

8. Automatischer Injektor nach Anspruch 7, **dadurch gekennzeichnet, dass** der zweite Hebel (43) elastisch verformbar ist, und dass der Schaft (6) einen Amboss (66) zum Halten des zweiten Hebels (43) während der Injektion aufweist, der den zweiten Hebel daran hindert, sich bei Kontakt des Riegels zu verformen, solange die Injektion nicht abgeschlossen ist.

9. Automatischer Injektor nach Anspruch 8, **dadurch gekennzeichnet, dass** der Amboss (66) am Schaft (6) eingerichtet ist, um mit dem Halten des zweiten Hebels (43) am Ende einer Injektion aufzuhören, wobei sich der zweite Hebel dann bei Kontakt des Riegels verformt, um die Verschiebung des Stutzens zu seiner Injektionsendposition freizugeben, und seine Ausgangsform durch elastische Rückstellung (F11) in eine Position (Figur 11) wieder annimmt, in der er sich dem Rückzug des Riegels des Stutzens entgegensetzt.

## Claims

1. An automatic injector (1), comprising :
- a cap (2), extending along a longitudinal axis (X1),
- a syringe (8) for injecting a medicament (P), which is positioned inside the cap and which includes a needle (84), a needle protector (86), a body (80) and a piston (82),
- a rod (6) for pushing the piston inside the syringe body, which is moved axially forward by a resilient load force (E16) exerted by a first spring (16), during an injection,
- an end-piece (10) for protecting the needle, which is movable axially around the needle and against a resilient load force (E14) exerted by a second spring (14), between a forward position (figure 2; figure 13) where it surrounds the needle and a withdrawn position (figure 8; figure 14), where the needle is exposed,
- means (41, 64; 106, 46) for blocking the forward movement of the rod,
**characterized in that**:
- the blocking means (41, 64; 106, 46) are configured to be deactivated when the end-piece (10) is moved toward its withdrawn position (figure 8; figure 14) or reaches that position, and to be reactivated during injection when the end-piece is moved toward its forward position (figure 2; figure 13) or arrives **in that** position, and
- the means (41, 64) for blocking the forward movement of the rod (6) comprise:
- a first lever (41) for blocking the movement of the rod, which is provided to block the forward movement of a rack (64) arranged on the push rod (6), the end-piece (10) comprising a cam (102) freeing the blocking action of first lever (41) on the rack (64), said cam driving the tilting (F2) of the first lever toward a free configuration of the rack when the end-piece arrives in the withdrawn position, or
- two elastic fins (106), which belong to the end-piece (10) and which are positioned on either side of the push rod (6), and two stops (46) blocking the elastic fins in a position (figure 13) where the elastic fins keep the rod in a vise between them.

2. The automatic injector according to claim 1, **characterized in that** the first lever (41) is elastically deformable and is deformed at the beginning of injection in contact with the cam (102), to tilt off the passage trajectory of the rack (64).

3. The automatic injector according to claim 2, **characterized in that** the first lever (41) is configured to resume its initial shape by elastic return (F5) if the end-piece returns toward its forward position during injection and **in that** this elastic return (F5) causes part (42b) of the lever to be housed in an indentation (640) of the rack (64), thus preventing the forward movement of the push rod (6).

4. The automatic injector according to claim 1, **characterized in that** the fins (106) each comprise a bearing surface (S108B) on a stop (46), which is inclined relative to a longitudinal axis (X62) of the rod (6) parallel to the longitudinal axis (X1) and that is complementary to a contact surface (S46) of the stop.

5. The automatic injector according to one of the preceding claims, **characterized in that** it further comprises means (43, 104; 69) for locking the end-piece (10) in a forward end of injection position (figure 11; figure 16), which opposes the withdrawal of the end-piece after injection.

6. The automatic injector according to claim 5, **characterized in that** the means for locking the end-piece comprise an elastic finger (69) belonging to the rod (6), which pivots (F16) at the end of injection by elastic return, to block the withdrawal of the fins (106) of the end-piece (10).

7. The automatic injector according to claim 5, **characterized in that** the locking means comprise a second lever (43) blocking the movement of the end-piece (10), which is provided to cooperate with a bolt (104) worn by the end-piece, this second lever opposing the withdrawal of the bolt of the end-piece when the end-piece is in the end of injection position (figure 11).

8. The automatic injector according to claim 7, **characterized in that** the second lever (43) is elastically deformable and **in that** the rod (6) comprises an anvil (66) maintaining the second lever (43) during the injection, which prevents the second lever from deforming in contact with the bolt as long as the injection is not complete.

9. The automatic injector according to claim 8, **characterized in that** the anvil (66) is arranged on the rod (6) to stop maintaining the second lever (43) at the end of injection, the second lever then deforming in contact with the bolt to free the movement of the end-piece toward its end of injection position and regaining its initial shape by elastic return (F11) in a position (figure 11) where it opposes the withdrawal of the bolt of the end-piece.
